# EUROPEAN PATENT APPLICATION

(11) **EP 2 796 486 A1**
(43) Date of publication of application: **29.10.2014**
(21) Application number: 12860012.9
(22) Date of filing: 04.10.2012
(51) Int. Cl.: C08J 3/075, C08J 5/18, A61F 2/28

(54) **HYDROGEL HAVING A DECOMPOSITION RATE CAPABLE OF BEING REGULATED IN SITU AND METHOD FOR MANUFACTURING SAME**

(30) Priority: 22.12.2011 KR 20110139849
(71) Applicant: Osstemimplant Co., Ltd., Seoul 153-759 (KR)
(72) Inventor: CHUNG, Yong Il, Busan 616-120 (KR); CHOI, Da Mi, Busan 611-080 (KR); SONG, Ju Dong, Busan 609-310 (KR); KANG, Eun Jung, Busan 608-020 (KR); EOM, Tae Gwan, Busan 612-030 (KR); CHOI, Kyoo Ok, Seoul 153-759 (KR)
(74) Representative: Caspary, Karsten
(86) International application number: PCT/KR2012/008017
(87) International publication number: WO 2013/094858

(57) **Abstract**

The present invention relates to a dental hydrogel and to a method for manufacturing same, and more particularly, to a hydrogel and to a method for manufacturing same wherein the hydrogel, which can be freely used during treatment irrespective of the shape of a bone defect region, has a decomposition rate capable of being regulated in situ and can thus be rapidly decomposed over a certain time, i.e., after the completion of bone regeneration. Furthermore, the present invention relates to a hydrogel membrane using the hydrogel which can be applied to a bone defect region irrespective of the shape thereof, and to a method for manufacturing same.

## Description

### [Technical Field]

The present invention relates to a hydrogel with adjustable biodegradation rate and a method for preparing the same and, more particularly, to a hydrogel which can be rapidly degraded due to its adjustable biodegradation rate after a certain time, i.e., after completion of bone regeneration, and a method for preparing the same. Moreover, the present invention relates to a hydrogel membrane which can be freely applied to a bone defect regardless of the shape of the bone defect and a method for preparing the same.

### [Background Art]

Sheet-type dental membranes have been mainly used in the past, but these sheet-type dental membranes should be cut to fit the shape of a bone defect, and thus the total time of surgical procedure is increased. Moreover, in the functional aspect, the sheet-type membrane is mainly composed of collagen and thus is rapidly absorbed into the body, and thus its space maintaining and soft tissue shielding functions deteriorate.

To solve these problems, U.S. Patent No. 6,306,922 discloses photopolymerizable hydrogel membranes, but these photopolymerizable hydrogel membranes have very low biodegradability due to high physical strength.

That is, in the case of the dental membrane, the membrane strength should be maintained until bone regeneration is complete and should be rapidly degraded after the completion of bone regeneration such that the membrane does not act as a barrier to the fusion of hard tissue and soft tissue. However, the photopolymerizable hydrogel membranes cannot be degraded at an appropriate time due to high physical strength.

### [Disclosure]

### [Technical Problem]

Accordingly, the present invention has been made to solve the above-described problems, and an object of the present invention is to provide a hydrogel which can be rapidly degraded due to its adjustable biodegradation rate after a certain time, i.e., after completion of bone regeneration, and a method for preparing the same. Moreover, another object of the present invention is to provide a hydrogel membrane which can be freely applied to a bone defect regardless of the shape of the bone defect and a method for preparing the same so as to overcome the inconvenience in the use of conventional sheet-type membranes.

### [Technical Solution]

To achieve the above objects, the present invention provides a hydrogel and a hydrogel membrane, each comprising polyethylene glycol (PEG) having a photopolymerizable functional group; a natural polymer additive forming an interpenetrating polymer network (IPN) together with photopolymerized PEG and having a viscosity and a biodegradation rate higher than those of the PEG; and a photopolymerization initiator generating a radical for initiating photopolymerization of the PEG.

Moreover, the present invention provides a method for preparing a hydrogel, the method comprising the steps of: i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG; ii) mixing the prepared first and second liquids in a cavity; and iii) forming a hydrogel in the form of an interpenetrating polymer network (IPN) by irradiating visible light to the mixed solution.

Furthermore, the present invention provides a method for preparing a hydrogel membrane, the method comprising the steps of: i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG; ii) mixing the prepared first and second liquids in a cavity; iii) shaping the mixed solution in the form of a membrane; and iv) forming a hydrogel in the form of an interpenetrating polymer network (IPN) by irradiation with visible light to the mixed solution.

In addition, the present invention provides a method for preparing a hydrogel membrane, the method comprising the steps of: i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG; ii) mixing the prepared first and second liquids in a cavity; iii) forming a hydrogel in the form of an interpenetrating polymer network (IPN) by irradiating visible light to the mixed solution; and iv) shaping the hydrogel in the form of a membrane.

The PEG may have a chemical formula of (-CH2CH2O-)n (n is an integer of 10 to 1,000) and comprise linear (2-arm) PEG, branded (4 or 8-arm) PEG, star-shaped (multi-arm) PEG or a combination thereof within the above range of the molecular weight.

Moreover, the photopolymerizable functional group may comprise at least one selected from the group consisting of acrylate, methacrylate, coumarin, thymine, and cinnamate.

The natural polymer additive may comprise at least one selected from the group consisting of carboxyl methyl cellulose, heparan sulfate, hyaluronic acid, collagen, chitosan, dextran, and alginate, and the molecular weight of the natural polymer additive may preferably be 100,000 to 10,000,000. Moreover, the photopolymerization initiator may generate a radical in response to visible light of a wavelength of 400 to 750 nm.

### [Advantageous Effects]

The dental hydrogel of the present invention can be freely applied to a bone defect regardless of the shape of the bone defect and has an interpenetrating polymer network (IPN) formed by PEG and a natural polymer additive, which makes it possible to adjust its biodegradation rate such that it can maintain the space maintaining and soft tissue shielding functions until a certain time, i.e., until bone regeneration is complete and can be rapidly degraded after the completion of bone regeneration.

### [Description of Drawings]

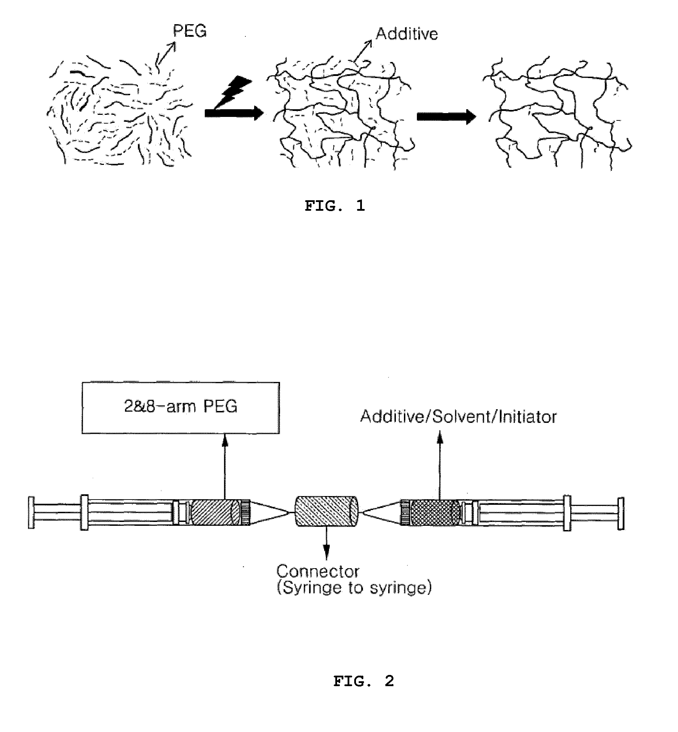

FIG. 1 is a conceptual diagram showing the formation and degradation of a hydrogel of the present invention.
FIG. 2 is a schematic diagram showing the mixing of a first liquid containing PEG and a second containing a natural polymer additive and a photopolymerization initiator in a cavity.

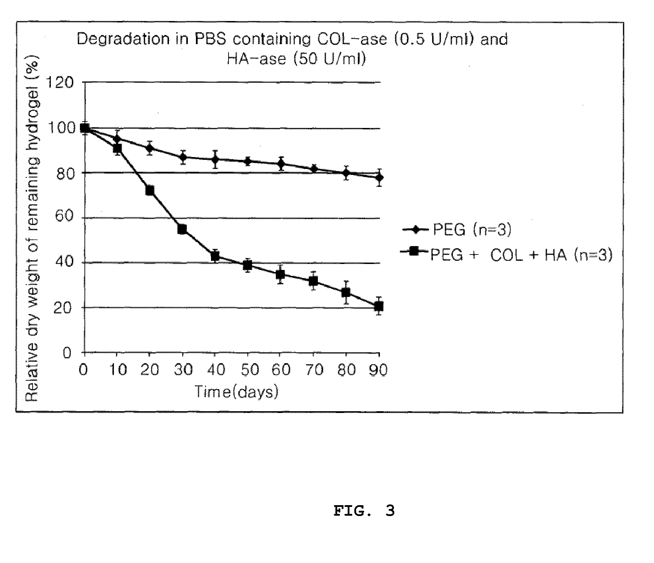

FIG. 3 shows the results of biodegradability evaluation of a hydrogel membrane in an in vivo-like environment (PEG: photopolymerizable PEG hydrogel membrane, PEG+COL+HA: photopolymerizable PEG hydrogel membrane in which collagen and hyaluronic acid are combined in an interpenetrating polymer network (IPN) according to the method of the present invention).

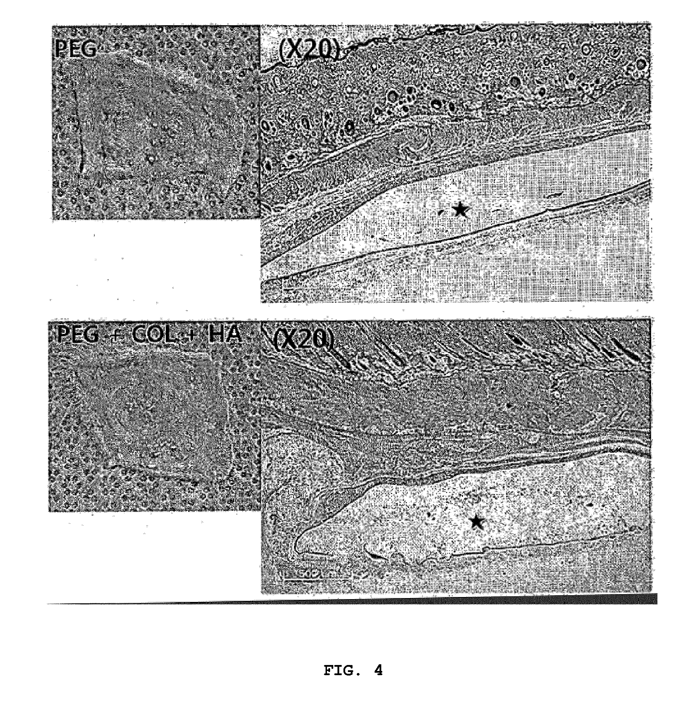

FIG. 4 shows the results of visual and histological evaluations of acute inflammatory response at 1 week of hydrogel membranes via subcutaneous implantation into the back of rats (★: subcutaneously grafted hydrogel membrane residue, PEG: photopolymerizable PEG hydrogel membrane, PEG+COL+HA: photopolymerizable PEG hydrogel membrane in which collagen and hyaluronic acid are combined in an IPN according to the method of the present invention).

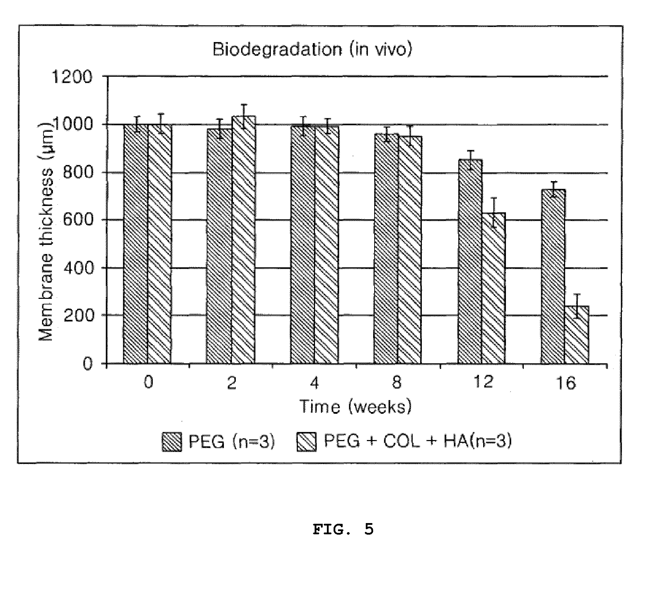

FIG. 5 shows the results of biodegradation evaluation of hydrogel membranes using rat subcutaneous implantation models (PEG: photopolymerizable PEG hydrogel membrane, PEG+COL+HA: photopolymerizable PEG hydrogel membrane in which collagen and hyaluronic acid are combined in an interpenetrating polymer network (IPN) according to the method of the present invention).

### [Mode for Invention]

Hereinafter, a hydrogel and a method for preparing the same in accordance with an aspect of the present invention will be described in detail with reference to the accompanying drawings.

A hydrogel of the present invention comprises polyethylene glycol (PEG) having a photopolymerizable functional group, a natural polymer additive forming an interpenetrating polymer network (IPN) together with photopolymerized PEG and having a viscosity and a biodegradation rate higher than those of the PEG, and a photopolymerization initiator generating a radical for initiating photopolymerization of the PEG, and has the interpenetrating polymer network (IPN) formed by mixing photopolymerized PED and the natural polymer additive.

A hydrogel using photopolymerization, which is used in a conventional membrane, has a very dense polymer network structure, in which several PEGs are cross-linked on one connecting vertex, and exhibits high physical strength. As a result, the conventional hydrogel is not degraded after completion of bone regeneration but remains in the body, and thus the membrane itself acts as a barrier to the fusion of hard tissue and soft tissue.

However, as shown in FIG. 1, in the case of a membrane to which the hydrogel of the present invention is applied, a natural polymer additive that forms the interpenetrating polymer network is first degraded at an appropriate time (target point), and thus the internal network structure of the hydrogel becomes loose. As a result, immune cells can easily infiltrate into the loose network, and thus the membrane is rapidly degraded.

That is, the natural polymer additive having a biodegradation rate higher than that of PEG leads the biodegradability, which thus increases the biodegradation rate of the hydrogel. Moreover, the addition of a natural polymer increases the affinity between the hydrogel and tissue, and thus when the hydrogel is used as a dental membrane, the fusion of hard tissue and soft tissue can be promoted.

The natural polymer additive may include various natural polymer materials having a biodegradation rate higher than that of PEG and may preferably include at least one selected from the group consisting of carboxyl methyl cellulose, heparan sulfate, hyaluronic acid, collagen, chitosan, dextran, and alginate. Here, the molecular weight of the natural polymer additive may preferably be in the range of 100,000 to 10,000,000.

Meanwhile, the PEG that forms the backbone of the polymer network is a polymer having a chemical formula of (-CH2CH2O-)n (n is an integer of 10 to 1,000), and linear (2-arm) PEG, branded (4 or 8-arm) PEG, and star-shaped (multi-arm) PEG may be used alone or in a combination thereof within the above range of the molecular weight.

The PEG component may have various photopolymerizable functional groups and may preferably have at least one functional group selected from the group consisting of acrylate, methacrylate, coumarin, thymine, and cinnamate.

Moreover, the photopolymerization initiator serves to generate a radical for initiating photopolymerization of the PEG and may preferably generate a radical in response to visible light of a wavelength of 400 to 750 nm.

The hydrogel may be prepared by the steps of: i) preparing a first liquid containing PEG and a second containing a natural polymer additive and a photopolymerization initiator; ii) mixing the prepared first and second liquids in a cavity; and iii) forming a hydrogel in the form of an interpenetrating polymer network (IPN) by irradiating visible light to the mixed solution.

Further, the membrane using the hydrogel may be prepared by the steps of: i) preparing a first liquid containing PEG and a second containing a natural polymer additive and a photopolymerization initiator; ii) mixing the prepared first and second liquids in a cavity; iii) shaping the mixed solution in the form of a membrane; and iv) applying visible light to the membrane, or by the steps of: i) preparing a first liquid containing PEG and a second containing a natural polymer additive and a photopolymerization initiator; ii) mixing the prepared first and second liquids in a cavity; iii) forming a hydrogel in the form of an interpenetrating polymer network (IPN) by irradiation with visible light to the mixed solution; and iv) shaping the hydrogel in the form of a membrane.

In detail, as shown in FIG. 2, the method for preparing a hydrogel according to the present invention comprises the steps of: preparing a first liquid containing PEG and a second containing a natural polymer additive and a photopolymerization initiator; and mixing the prepared first and second liquids in a cavity. Here, the natural polymer additive imparts viscosity to the mixed solution to prevent the mixed solution from flowing to the periphery, and thus it is preferable that the natural polymer additive has a viscosity higher than that of PEG.

Since the mixed solution has a viscosity sufficient to prevent it from flowing to the periphery, it is possible to freely shape the mixed solution in the form of an appropriate membrane by applying the mixed solution to a mold corresponding to the shape of a bone defect using an injectable container, etc., or by directly applying the mixed solution to the bone defect of a mammalian bone defect. The thus shaped membrane is polymerized by irradiation with visible light to finally form a hydrogel membrane in the form of an interpenetrating polymer network (IPN).

For the use of the hydrogel of the present invention as a dental material, various methods such as a method of using a paste container, a direct shaping method, a method of using a mold, etc. may be used in addition to the above-mentioned method of using an injectable container without departing from the gist of the present invention.

Data related to the biodegradability and biosafety of the hydrogel membrane prepared in the above manner can be found in FIGS 3 to 5. In FIG. 3, in vivo-like enzyme reaction conditions were created and the biodegradabilities of hydrogel membranes were compared and evaluated. In the case of an existing PEG hydrogel membrane, the degradation by hydrolysis and enzyme reaction is relatively low due to a dense polymer network, and thus the membrane is hardly degraded even after 90 days, but mostly remains. On the contrary, in the case of a PEG+COL+HA hydrogel membrane in which collagen (COL) and hyaluronic acid (HA) form the IPN structure together with cross-linked PEG, its biodegradability is similar to that of a PEG hydrogel membrane, but after collagen and hyaluronic acid are degraded by enzyme reaction, its internal network structure becomes loose, and the swelling degree is relatively increased. As a result, the internal degradation by hydrolysis is activated, and thus the degradation of the membrane is increased. The actual in vivo conditions are not an environment where an excessive amount of buffer is present, like the above experimental conditions, and thus it cannot be said that the results represent the biodegradation behavior, but can be used as a basis for predicting the tendency.

FIG. 4 shows images of the results of acute inflammatory response at 1 week after in vivo implantation of hydrogel membranes. Initial acute inflammatory response was not observed in all of the PEG and PEG+COL+HA hydrogel membrane systems, and specific inflammatory response was also not observed through histological analysis other than common tissue response to biomaterials. Therefore, it was found that the hydrogel membrane of the present invention satisfied the biocompatibility.

FIG. 5 shows the results of biodegradation evaluation of hydrogel membranes having histocompatibility. The in vivo degradation was not the same as the results of FIG. 3, but the difference in degradation between two hydrogels was similarly observed after a certain time. In the case of the hydrogel membrane mainly composed of PEG, about 70% of the membrane remained up to 16 weeks. However, in the case of the hydrogel membrane of the present invention, the biodegradability was rapidly increased after 8 weeks, about 60% of the membrane was biodegraded up to 12 week, and most of the membrane was biodegraded at 16 weeks. Therefore, it was possible to adjust the degradation rate so as to accelerate the biodegradability after a certain time by combining collagen and hyaluronic acid with PEG in the IPN structure, while the photopolymerizable hydrogel composed of PEG only was slowly biodegraded linearly.

Meanwhile, in another embodiment of the present invention, the hydrogel membrane may be prepared by curing the mixed solution of the first liquid and the second liquid in the form of a hydrogel by direct irradiation of visible light and then applying the hydrogel to a bone defect at an appropriate pressure.

The present invention is not limited to the above-described specific embodiments and description, and it will be appreciated by those skilled in the art that changes may be made in these embodiments without departing from the principles and spirit of the invention, the scope of which is defined in the appended claims and their equivalents.

## Claims

1. A hydrogel comprising:
polyethylene glycol (PEG) having a photopolymerizable functional group;
a natural polymer additive forming an interpenetrating polymer network (IPN) together with photopolymerized PEG and having a viscosity and a biodegradation rate higher than those of the PEG; and
a photopolymerization initiator generating a radical for initiating photopolymerization of the PEG.

2. The hydrogel of claim 1, wherein the PEG has a chemical formula of (-CH2CH2O-)n (n is an integer of 10 to 1,000) and comprises linear (2-arm) PEG, branded (4 or 8-arm) PEG, star-shaped (multi-arm) PEG or a combination thereof within the above range of the molecular weight.

3. The hydrogel of claim 1, wherein the photopolymerizable functional group comprises at least one selected from the group consisting of acrylate, methacrylate, coumarin, thymine, and cinnamate.

4. The hydrogel of claim 1, wherein the natural polymer additive comprises at least one selected from the group consisting of carboxyl methyl cellulose, heparan sulfate, hyaluronic acid, collagen, chitosan, dextran, and alginate.

5. The hydrogel of claim 4, wherein the molecular weight of the natural polymer additive is 100,000 to 10,000,000.

6. The hydrogel of claim 1, wherein the hydrogel is applied to a bone defect.

7. A method for preparing a hydrogel, the method comprising the steps of:
i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG;
ii) mixing the prepared first and second liquids in a cavity; and
iii) forming a hydrogel in the form of an interpenetrating polymer network (IPN) by irradiating visible light to the mixed solution.

8. The method of claim 7, wherein the photopolymerization initiator generates a radical in response to visible light of a wavelength of 400 to 750 nm.

9. The method of claim 7, wherein the PEG has a chemical formula of (-CH2CH2O-)n (n is an integer of 10 to 1,000) and comprises linear (2-arm) PEG, branded (4 or 8-arm) PEG, star-shaped (multi-arm) PEG or a combination thereof within the above range of the molecular weight.

10. The method of claim 7, wherein the photopolymerizable functional group comprises at least one selected from the group consisting of acrylate, methacrylate, coumarin, thymine, and cinnamate.

11. The method of claim 7, wherein the natural polymer additive comprises at least one selected from the group consisting of carboxyl methyl cellulose, heparan sulfate, hyaluronic acid, collagen, chitosan, dextran, and alginate.

12. The method of claim 7, wherein the molecular weight of the natural polymer additive is 100,000 to 10,000,000.

13. A hydrogel membrane comprising:
polyethylene glycol (PEG) having a photopolymerizable functional group;
a natural polymer additive forming an interpenetrating polymer network (IPN) together with photopolymerized PEG and having a viscosity and a biodegradation rate higher than those of the PEG; and
a photopolymerization initiator generating a radical for initiating photopolymerization of the PEG.

14. The hydrogel membrane of claim 13, wherein the PEG has a chemical formula of (-CH2CH2O-)n (n is an integer of 10 to 1,000) and comprises linear (2-arm) PEG, branded (4 or 8-arm) PEG, star-shaped (multi-arm) PEG or a combination thereof within the above range of the molecular weight.

15. The hydrogel membrane of claim 13, wherein the photopolymerizable functional group comprises at least one selected from the group consisting of acrylate, methacrylate, coumarin, thymine, and cinnamate.

16. The hydrogel membrane of claim 13, wherein the natural polymer additive comprises at least one selected from the group consisting of carboxyl methyl cellulose, heparan sulfate, hyaluronic acid, collagen, chitosan, dextran, and alginate.

17. The hydrogel membrane of claim 16, wherein the molecular weight of the natural polymer additive is 100,000 to 10,000,000.

18. The hydrogel membrane of claim 13, wherein the hydrogel membrane is applied to a bone defect.

19. A method for preparing a hydrogel membrane, the method comprising the steps of:
i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG;
ii) mixing the prepared first and second liquids in a cavity;
iii) shaping the mixed solution in the form of a membrane; and
iv) forming a hydrogel in the form of an interpenetrating polymer network (IPN) by irradiation with visible light to the mixed solution.

20. A method for preparing a hydrogel membrane, the method comprising the steps of:
i) preparing a first liquid containing polyethylene glycol (PEG) having a photopolymerizable functional group and a second liquid containing a natural polymer additive having a viscosity and a biodegradation rate higher than those of the PEG and a photopolymerization initiator generating a radical for initiating photopolymerization of the PEG;
ii) mixing the prepared first and second liquids in a cavity;
iii) forming a hydrogel in the form of an interpenetrating polymer network (IPN) by irradiating visible light to the mixed solution; and
iv) shaping the hydrogel in the form of a membrane.

21. The method of claim 19 or 20, wherein the photopolymerization initiator generates a radical in response to visible light of a wavelength of 400 to 750 nm.

22. The method of claim 19 or 20, wherein the PEG has a chemical formula of (-CH2CH2O-)n (n is an integer of 10 to 1,000) and comprises linear (2-arm) PEG, branded (4 or 8-arm) PEG, star-shaped (multi-arm) PEG or a combination thereof within the above range of the molecular weight.

23. The method of claim 19 or 20, wherein the photopolymerizable functional group comprises at least one selected from the group consisting of acrylate, methacrylate, coumarin, thymine, and cinnamate.

24. The method of claim 19 or 20, wherein the natural polymer additive comprises at least one selected from the group consisting of carboxyl methyl cellulose, heparan sulfate, hyaluronic acid, collagen, chitosan, dextran, and alginate.

25. The method of claim 19 or 20, wherein the molecular weight of the natural polymer additive is 100,000 to 10,000,000.
